Europäisches Patentamt

European Patent Office

Office européen des brevets

(11) Publication number: **0 221 096**
**B1**

# EUROPEAN PATENT SPECIFICATION

(45) Date of publication of patent specification: 26.09.90

(21) Application number: 86902348.1

(22) Date of filing: 08.04.86

(86) International application number:
PCT/DK86/00032

(87) International publication number:
WO 86/05954 23.10.86 Gazette 86/23

(51) Int. Cl.⁵: **A 23 L 1/304,** A 23 L 1/237,
A 61 K 33/00

(54) MINERAL SALT PREPARATION AND METHOD OF PRODUCING SAME.

(30) Priority: 09.04.85 DK 1563/85
29.04.85 DK 1902/85

(43) Date of publication of application:
13.05.87 Bulletin 87/20

(45) Publication of the grant of the patent:
26.09.90 Bulletin 90/39

(84) Designated Contracting States:
AT BE CH DE FR GB IT LI NL SE

(56) References cited:
WO-A-85/02324
DE-A-3 008 171
DE-A-3 201 405
DE-B-2 060 601
SE-B- 440 019
US-A-4 107 346

(73) Proprietor: SUNPOL CONSULT ApS
Heslehoj Alle 9
DK-2900 Hellerup (DK)

(72) Inventor: SUNDIEN, Gunnar, Olof
Heslehoj Alle 9
DK-2900 Hellerup (DK)

(74) Representative: Sundien, Gunnar et al
HOFMAN-BANG & BOUTARD A/S Adelgade 15
DK-1304 Copenhagen K (DK)

Courier Press, Leamington Spa, England.

# EP 0 221 096 B1

**Description**

The invention relates to a mineral salt composition for use as an additive for food or nutrients or as a medicament. The invention further relates to a method of producing the mineral salt composition from sea water. The term 'sea water' is used herein in the sense of ocean water.

While, in ancient times, substantially untreated and naturally occurring nutrients were used as food for man, people in the industrialized world are now, to an increasing extent, using refined food, the nutritional value of which is reduced because vitamins and minerals have been more or less removed or deteriorated during refining processes or other treatments. The consequence of this can be faulty nutrition and deficiency diseases.

In recent times, it has been proved that many minerals are essential components of the food because they act as coenzymes in combination with many enzymes. In some cases the cations from mineral salts form complex compounds with certain enzymes. These so-called metallo-enzymes exist in an active and an inactive state, depending on different control mecanisms that regulate the complex formation. In order to secure satisfactory function of these processes, said compounds must be present in optimal concentration intervals. Many of these compounds are found in very low concentrations and therefore they are often called trace elements.

Examples of essential trace elements are V, Cr, Mn, Co, Cu, Zn, As, Mo and Se, the activity of which is known, but many other elements may be and probably are essential.

Attempts have been mde to eliminate the risk of deficiency diseases due to low content of trace elements in the food by supply of minerals to the organism as food supplement. Thus, in DK-507/73 is disclosed a preparation containing 0,1—0,0001% of several trace elements.

In this way it is difficult, however, to secure optimal supply of all trace elements. Partly, the correct optimal concentration of all elements are hardly known, and partly it is difficult to dose exact amounts of minerals in very low concentrations. Further, an excessive amount of certain elements might expel other trace elements or block the activity of these. An excess of Cd or Ca will, for example, cause deficiency of Zn, excess of Mn will block for Mg, and excess of Zn or Mo may counteract Cu.

In order to obtain a desired taste or increase stability, large amounts of salt as pure sodium chloride are often added to the food. Certain nutrients, such as fish products are often preserved in salt brine, the main constituent of which is pure sodium chloride.

Further, it is common practice, during consumption of food and in order to obtain a desired taste, to add table salt to the food.

In this way, many people consume a larger amount of salt than needed for covering their natural requirement. The excess of salt must be secreted from the organism through the kidneys, which might thus be overloaded. Further, a too high salt intake might change the natural electrolyte balance of the organism, which might have serious consequences for the health. A frequent result of exaggerated salt intake is hypertension and circulation diseases, such as arteriosclerosis, brain haemorrhage and blood clots. Prolonged salt intake is also under suspicion as a cause for stomach cancer.

The electrolyte balance, that secures a correct electrical potential over the cell membranes, is primarily determined by the concentrations of sodium, potassium and magnesium ions. The normal concentrations of these cations, expressed in meqv/l, is shown in the following table:

TABLE

| Cation | Blood-serum | Intracellular liquid in | |
| | | Erythrocyte | Muscle cell |
| --- | --- | --- | --- |
| $Na^+$ | 142 | 30 | 5 |
| $K^+$ | 5—5,5 | 128 | 150 |
| $Mg^{++}$ | 3—3,5 | 6 | 40 |

Due to the concentration differences between the intracellular and extracellular liquids the electrical potential over the cell membrane is about 60 mV for erythrocytes and connective tissue and 90—120 mV for muscle tissue. Persons having low metabolism or other abnormal conditions can have other ion concentrations and substantially lower membrane potentials than the above mentioned. Such conditions are often accompanied with abnormal high content of liquid in the tissues, resulting in edema.

The risk connected with consumption of large amount of salt as pure sodium chloride has been known for a long time, and for that reason it has been proposed to use salt mixtures containing substantial amounts of sodium, magnesium and calcium salts. Thus, USP 1 998 179 suggests that an optimal physiologically balanced salt mixture contains Na, K, Ca and Mg in a molecular proportion of about 100:5:2,5:2,5.

The use of this known salt mixture will only solve the problem of maintaining the electrolyte balance, which gives a correct potential over the cell membrane. On the other hand, no trace element is supplied, and the optimal amounts of trace elements can not be secured by supplement of known food additives containing trace elements. As mentioned above, it is practically difficult to dose trace elements correctly in very small amounts, and further, the required amounts of trace elements also depend on the total amount of supplied salt.

Even when using a salt mixture containing Na, K and Mg in optimal concentrations, a tendency to overdosing the salt intake still exists. When the kidneys secrete this excess, a secretion of all trace elements will simultaneously occur in amounts proportional to the secreted salt.

An object of the present invention is to provide for a mineral salt composition that supplies Na, K, Ca and Mg in optimal amounts and also secures supply of all trace elements in the correct balanced proportion as needed by the organism.

Another object of the invention is to provide a simple and economic method of manufacturing this mineral salt composition.

The mineral salt composition in accordance with the invention is characterised by a content of soluble mineral salts in substnatially the same relative proportion as found in sea (i.e. ocean) water, except for a reduced amount of sodium chloride, and takes the form of a dry, homogenous, freely flowing powder or a granulate, suitable as a table salt, or which can be comprssed to tablets containing unit doses as a food additive or medicament, if desired containing usual diluents and/or additives.

The method of the invention is characterised in that sea water is evaporated until a substantial amount of sodium chloride is precipitated, after which the mother lye is isolated, evaporated and dried. The evaporation and drying processes can be performed in any way, such as drying and drum drying, which leads to a homogeneous particulate or granular product.

As starting material for the method of manufacturing mother lye can be used which is obtained as a byproduct from the recovery of sea salt in tropical or subtropical coastal areas, such as Portugal or Spain.

Sea salt is usually recovered in large open basins in which the sea water is introduced and subjected to sun radiation. In a first step, concentration occurs and then sodium chloride crystals are separated, while highly soluble potassium and magnesium salts and all the trace elements remain in solution. The evaporation proceeds as long as the sodium chloride crystals are sufficiently pure. When other salts than sodium chloride begin to crystallize, the evaporation is interrupted and the mother lye is returned to the sea as a waste product. This mother lye is especially suitable as a starting material for the process of the invention. A special advantage of the process is that a waste product, which is worthless per se, is utilized.

It has been shown that the content of the inorganic components of the sea water corresponds very closely to the content of the fluids in the human organism, except that the amount of sodium chloride is relatively much higher in sea water than in the organism. This is in accordance with the fact that mans ancestors in early stages of the evolution were living in sea water and adapted their enzyme system to this environment.

The content of several elements in sea water compared to human cells is illustrated in the diagram of the accompanied drawing (ref: Sandoz Bulletin 51, 1979), where the abscissa represents the elements of the periodic system and the ordinate indicates the logarithm of the number of ions per nl for sea water and per cell for the organism.

It is seen that a surprising correlation exists between the relative concentrations of the two media. The biggest difference is that Na and Cl are found in substantially higher concentrations in sea water than in the organism. When a substantial part of the sodium chloride is removed from the sea water, a product is provided with almost the same composition as the fluid in the human cells.

Due to the special composition of the mineral salt of the invention the risk of overdosing is reduced. When excess of sodium chloride is excreted through the kidneys potassium, magnesium and all essential trace elements will also be excreted, namely in the same relative proportions as they are added to the organism, so that neither a displacement of the electrolyte balance nor a depletion of the trace elements occur.

The use of the mineral salt of the invention is accordingly suitable for the treatment of deficiency diseases and preventing diseases which are caused by a wrong mineral balance due to excessive use of pure salt having a low content of other salts than sodium chloride.

DE—A—3,008,171 does not refer to a sea salt, recovered from the oceans. In spite of the word "Meersalz", the product is derived from the "Wasser des Toten Meeres", which is a lake having neither outlet nor inlet from the oceans.

For that reason, the water contains mineral salts having a totally different composition from that of the water in the oceans. In the patent description, page 7, last paragraph is indicated that the water is a "carnallithaltige Salzkoncentrat" which is "nicht unmittelbar als Speisesalz zu verwenden". In order to produce an acceptable table salt from this solution, it is proposed to add 70—90% pure sodium chloride.

The product described in DE 3201405 comprises 4 different elements, none of which is sodium that are added to distilled water. This product would not taste like a table salt. It is also proposed as a component of drinking water of low concentration.

The product described in DE 2060601 is a mixture of 5—12 parts potassium to 1 part sodium. It may also contain Ca, Mg and some trace elements. The product would be useless as a table salt due to the special taste. It is intended as a mineral supplement or food additive, probably administered as tablets or pills.

3

The product of US 4,107,346 comprises 92—93.1% sodium chloride and is not a reduced sodium salt.

GB 2,015,863 covers a mixture of preferably 50—65% sodium 20—40% potassium and 5—20% magnesium. It has no content of trace elements. Insofar as the product is said in Claim 13 to be "based on rock or sea salt" it is clear that this means that the potassium and magnesium are added, as described on page 6, lines 107—114, to sodium chloride in the form of rock salt or crystallised from sea water in the conventional manner.

It is not obvious, from this or any of the other citations, to provide a composition as claimed with all the mineral constituents of sea water (other than sodium chloride) in substantially unchanged proportions for use as a table salt or the like.

The invention is illustrated by the following examples:

Example 1

As a raw material was used a saturated mineral salt solution produced by partial evaporation of sea water and removal of precipitated crystalline sodium chloride. The density of the solution was 1.21 and the content of dry material was about 30 weight%.

The solution was spray dried at a temperature of about 140°C. A fine crystalline freely flowing powder was produced the analysis of which was as follows:

| | |
|---|---|
| Sodium | 28% |
| Potassium | 1,5% |
| Magnesium | 5,3% |
| Lead | below 4 ppm |
| Cadmium | below 0,4 ppm |
| Mercury | below 0,02 ppm |

The salt mixture can be used as a table salt.

Example 2

A mineral salt solution as described in Example 1 was dried on a rotary drum under vacuum and the dried product was crushed to a coarse crystalline granulate, suitable as an additive for nutrients.

Example 3

A mineral salt solution as described in Example 1 was mixed with the same amount of calcium carbonate and granulated and simultaneously dried. The granulate was compressed on a tabletting machine to form tablets weighing 200 mg, suitable for oral administration as a disease preventing additive.

Example 4

A mineral salt solution as described in Example 1 was introduced in gelatine capsules corresponding to 200 mg dry material per capsule. The capsules are suitable as a food additive for oral administration.

Example 5

The mineral salt solution described in Example 1 was evaporated to half the volume and the precipitated crystalline sodium chloride was filtered off. 1,5 g of the mother lye was mixed with one litre of deionized water and the solution was saturated with carbon dioxide. The produced mineral beverage had an acceptable taste.

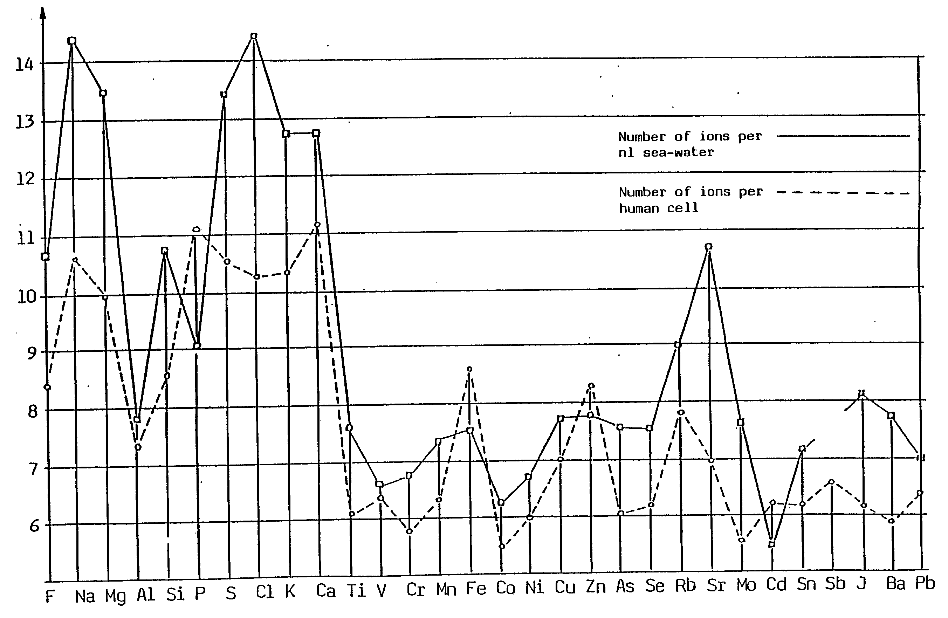

**Claims**

1. A mineral salt composition, comprising a dry, homogenous particulate or granulated mixture of soluble mineral salts evenly distributed as a homogenous mixture and containing the mineral salts in substantially the same relative proportions as in ocean water, except for a reduced content of sodium chloride.

2. A mineral salt composition for use as a tablet salt or as a food additive, comprising a dry, homogeneous particulate or granulated mixture of soluble mineral salts evenly distributed as a homogenous mixture and containing the mineral salts in substantially the same relative proportions as in ocean water, except for a reduced content of sodium chloride.

3. Table salt consisting of a freely flowing powder or granulate having the composition as defined in Claim 2.

4. Food additive, consisting of the mineral salt mixture of Claim 1.

5. Food additive in accordance with Claim 4, in the form of a tablet for oral administration containing

EP 0 221 096 B1

the mineral salt composition in accordance with Claim 1 and, if desired, containing usual diluents and/or additives.

6. A medicament for prevention or treatment of deficiency diseases, containing a mineral salt mixture according to Claim 1.

7. A method of producing a mineral salt composition, as defined in Claim 1 or Claim 2, comprising the steps of:—

(a) evaporating ocean water until a part of the sodium chloride dissolved in the ocean water is separated;

(b) isolating the mother lye from separated crystalline sodium chloride; and

(c) drying the isolated mother lye under such conditions that a dry, homogenous particulate or granulated mixture of the remaining mineral salt fraction is formed.

8. A method in accordance with Claim 7, wherein the ocean water is evaporated to such an extent that the remaining mother lye, after removal of the precipitated crystalline sodium chloride, contains at least 1 weight percent potassium and at least 4 weight percent magnesium, based on dissolved dry substance, after which the mother lye is isolated and evaporated to form a dry crystalline powder.

9. A method in accordance with Claim 7 or Claim 8, wherein the remaining mother lye, after removal of precipitated crystalline sodium chloride, is subsequently dried in a spray drying apparatus or a drum dryer.

10. A method in accordance with any one of Claims 7 to 9, wherein the dry mineral salt composition is compressed, if desired together with usual diluents and/or additives, to form tablets for use as a food additive or medicament.

**Patentansprüche**

1. Mineralsalzmischung, bestehend aus einem trockenen, homogenen, teilchenförmigen oder granulierten Gemisch löslicher Mineralsalze, gleichförmig als homogenes Gemisch verteilt und die Mineralsalze in praktisch den gleichen relativen Anteilen wie in Meerwasser enthaltend, mit der Ausnahme eines verminderten Gehaltes an Natriumchlorid.

2. Mineralsalzmischung zur Verwendung als Tafelsalz oder Lebensmittelzusatz, bestehend aus einem trockenen, homogenen, teilchenförmigen oder granulierten Gemisch löslicher Mineralsalze, gleichförmig als homogenes Gemisch verteilt und die Mineralsalze in praktisch den gleichen relativen Anteilen wie in Meerwasser enthaltend, mit der Ausnahme eines verminderten Gehaltes an Natriumchlorid.

3. Tafelsalz, bestehend aus einem rieselfähigen Pulver oder Granulat mit der Zusammensetzung gemäss Anspruch 2.

4. Lebensmittelzusatz, bestehend aus der Mineralsalzmischung gemäss Anspruch 1.

5. Lebensmittelzusatz nach Anspruch 4, in Form von Tabletten zur oralen Verabreichung, enthaltend die Mineralsalzmischung gemäss Anspruch 1 und, wenn gewünscht, übliche Streckmittel und/oder Zusätze.

6. Medikament zur Verhütung oder Behandlung von Mangelkrankheiten, enthaltend eine Mineralsalzmischung nach Anspruch 1.

7. Verfahren zur Herstellung einer Mineralsalzmischung nach Anspruch 1 oder 2, umfassend folgende Schritte:

a) Verdampfung von Meerwasser, bis sich ein Teil des im Meerwasser gelösten Natriumchlorids ausgeschieden hat,

b) Abtrennung der Mutterlauge vom Ausgeschiedenen Natriumchloride, und

c) Trocknung der abgetrennten Mutterlauge unter Bedingungen, bei denen ein trockenes, gleichförmiges, teilchen- oder kornförmiges Gemisch der verbleibenden Mineralsalz fraktion gebildet wird.

8. Verfahren nach Anspruch 7, wobei das Meerwasser sowie eingedampft wird, bis die verbleibende Mutterlauge nach Entfernung des ausgefallenen kristallinen Natriumchlorids mindestens 1 Gew.-% Kalium und mindestens 4 Gew.-% Magnesium enthält, bezogen auf gelöste Trockensubstanz, wonach die Mutterlauge abgetrennt und unter Bildung eines trockenen kristallinen Pulvers verdampft wird.

9. Verfahren nach Anspruch 7 oder 8, worin die verbliebene Mutterlauge nach Entfernung des ausgefallenen kristallinen Natriumchlorids in einem Sprühtrockner oder Trommeltrockner getrocknet wird.

10. Verfahren nach einem der Ansprüche 7 bis 9, worin die trockene Mineralsalzmischung gegebenenfalls zusammen mit üblichen Streckmitteln und/oder Zusätzen zu Tabletten zwecks Verwendung als Lebensmittelzusatz oder Medikament verpresst wird.

**Revendications**

1. Composition de sels minéraux, comprenant un mélange sec et homogène de particules ou de granulés de sels minéraux solubles, uniformément distribués sous forme de mélange homogène et contenant les sels minéraux dans substantiellement les mêmes proportions relatives que dans l'eau de mer, hormis une teneur réduite de chlorure de sodium.

2. Composition de sels minéraux pour l'utilisation comme sel de table ou additif alimentaire, comprenant un mélange sec et homogène de particules ou de granulés de sels minéraux solubles, uniformément distribués sous forme de mélange homogène et contenant les sels minéraux dans substantiellement les mêmes proportions relatives que dans l'eau de mer, hormis une teneur réduite de chlorure de sodium.

3. Sel de table, consistant en une poudre ou un granulé s'écoulant librement, ayant la composition telle que définie dans la revendication 2.

4. Additif alimentaire, consistant en un mélange de sels minéraux selon la revendication 1.

5. Additif alimentaire selon la revendication 4 sous forme d'un comprimé pour l'administration orale, contenant la composition de sels minéraux selon la revendication 1 et, si désiré, des diluants et/ou additifs usuels.

6. Médicament pour la prévention ou le traitement de maladies par carence, contenant un mélange de sels minéraux selon la revendication 1.

7. Procédé de fabrication d'une composition de sels minéraux selon la revendication 1 ou 2, comprenant les étapes suivantes:

(a) évaporation d'eau de mer jusqu'à séparation d'une partie du chlorure de sodium dissous dans l'eau de mer;

(b) isolation de la lessive-mère du chlorure sodium cristallisé séparé; et

(c) séchage de la lessive-mère isolée dans des conditions aptes à former un mélange particulaire ou granulé sec et homogène de la fraction resistante de sels minéraux.

8. Procédé selon la revendication 7 dans lequel l'eau de mer est évaporée à un tel degré que la lessive-mère restante contient, après séparation du chlorure de sodium cristallisé précipité, ou moins 1% en poids de potassium et au moins 4% en poids de magnésium, basés sur la matière sèche en solution, sur quoi la lessivemère est isolée et évaporée pour donner une poudre cristalline sèche.

9. Procédé selon la revendication 7 ou 8 dans lequel la lessive-mère restante, après l'enlèvement du chlorure de sodium cristallin précipité, est ensuite séchée dans un séchoir à pulvérisation ou un séchoir à tambour.

10. Procédé selon l'une quelconque des revendications 7 à 9, dans lequel la composition de sels minéraux secs est comprimée, si désiré avec des diluants et/ou additifs usuels, pour donner des comprimés pour l'utilisation comme additif alimentaire ou médicament.